# EUROPEAN PATENT APPLICATION

(11) **EP 1 241 177 A1**
(43) Date of publication of application: **18.09.2002**
(21) Application number: 02075925.4
(22) Date of filing: 11.03.2002
(51) Int. Cl.: C07K 14/03, C12N 7/04, C12N 15/86, A61K 39/245

(54) **Recombinant infectious laryngotracheitis virus vaccine**

(30) Priority: 15.03.2001 EP 01200975
(71) Applicant: Akzo Nobel N.V., 6824 BM Arnhem (NL)
(72) Inventor: Claessens, Johannes Antonius Joseph, 5831 WL Boxmeer (NL); Fuchs, Walter, 17498 Insel Riems (DE)
(74) Representative: Mestrom, Joannes Jozef Louis

(57) **Abstract**

The present invention provides an attenuated ILT virus that is able to induce protection against ILT in chickens. The new vaccine strain is not able to express the native UL0 protein of ILTV. The new ILTV vaccine virus can also be used as a vector for genes of other avian pathogens.

SEQUENCE LISTING

<110> AKZO Nobel NV

<120> Recombinant infectious laryngotracheitis virus vaccine

<160> 2

<170> PatentIn version 3.1

<210> 1

<211> 1711

<212> DNA

<213> Avian influenza virus

<220>

<221> cDNA

<222> (1)..(1711)

<223> isolate A/Italy/445/99 (H7/N1)

<220>

<221> CDS

<222> (11)..(1705)

<223>

<400> 1
gggatacaaa atg aac act caa atc ctg gta ttc gct ctg gtg gcg atc 49
Met Asn Thr Gln Ile Leu Val Phe Ala Leu Val Ala Ile
1               5                   10

att ccg aca agt gca gac aaa atc tgc ctt ggg cat cat gcc gtg tca 97
Ile Pro Thr Ser Ala Asp Lys Ile Cys Leu Gly His His Ala Val Ser
    15                  20                  25

aac ggg act aaa gta aac aca tta act gaa aga gga gtg gaa gtc gtt 145
Asn Gly Thr Lys Val Asn Thr Leu Thr Glu Arg Gly Val Glu Val Val
30                  35                  40                  45

aat gca act gaa acg gtg gaa cga aca aac gtc ccc agg atc tgc tca    193
Asn Ala Thr Glu Thr Val Glu Arg Thr Asn Val Pro Arg Ile Cys Ser
                50                  55                  60

aaa ggg aaa agg aca gtt gac ctc ggt caa tgt gga ctt ctg gga aca    241
Lys Gly Lys Arg Thr Val Asp Leu Gly Gln Cys Gly Leu Leu Gly Thr
            65                  70                  75

atc act ggg cca ccc caa tgt gac cag ttc cta gaa ttt tca gcc gat    289
Ile Thr Gly Pro Pro Gln Cys Asp Gln Phe Leu Glu Phe Ser Ala Asp
        80                  85                  90

cta att att gag agg cga gaa gga agt gat gtc tgt tat cct ggg aaa    337
Leu Ile Ile Glu Arg Arg Glu Gly Ser Asp Val Cys Tyr Pro Gly Lys
    95                  100                 105

ttc gtg aat gaa gaa gct ctg agg caa att ctc agg gag tca ggc gga    385
Phe Val Asn Glu Glu Ala Leu Arg Gln Ile Leu Arg Glu Ser Gly Gly
110                 115                 120                 125

att gac aag gag gca atg gga ttc aca tac agc gga ata aga act aat    433
Ile Asp Lys Glu Ala Met Gly Phe Thr Tyr Ser Gly Ile Arg Thr Asn
                130                 135                 140

gga aca acc agt aca tgt agg aga tca gga tct tca ttc tat gca gag    481
Gly Thr Thr Ser Thr Cys Arg Arg Ser Gly Ser Ser Phe Tyr Ala Glu
            145                 150                 155

atg aaa tgg ctc ctg tca aac aca gac aat gct gct ttc ccg cag atg    529
Met Lys Trp Leu Leu Ser Asn Thr Asp Asn Ala Ala Phe Pro Gln Met
        160                 165                 170

act aag tca tac aaa aac aca agg aaa gac cca gct ctg ata ata tgg    577
Thr Lys Ser Tyr Lys Asn Thr Arg Lys Asp Pro Ala Leu Ile Ile Trp
    175                 180                 185

ggg atc cac cat tcc gga tca act aca gaa cag acc aag cta tat ggg 625
Gly Ile His His Ser Gly Ser Thr Thr Glu Gln Thr Lys Leu Tyr Gly
190                 195                 200                 205

agt gga aac aaa ctg ata aca gtt ggg agt tct aat tac caa cag tcc 673
Ser Gly Asn Lys Leu Ile Thr Val Gly Ser Ser Asn Tyr Gln Gln Ser
                210                 215                 220

ttt gta ccg agt cca gga gag aga cca caa gtg aat ggc caa tct gga 721
Phe Val Pro Ser Pro Gly Glu Arg Pro Gln Val Asn Gly Gln Ser Gly
            225                 230                 235

aga att gac ttt cat tgg ctg atg cta aac ccc aat gac aca gtc act 769
Arg Ile Asp Phe His Trp Leu Met Leu Asn Pro Asn Asp Thr Val Thr
        240                 245                 250

ttc agt ttc aat ggg gcc ttc ata gct cca gac cgt gca agt ttt ctg 817
Phe Ser Phe Asn Gly Ala Phe Ile Ala Pro Asp Arg Ala Ser Phe Leu
    255                 260                 265

aga ggg aag tct atg ggg att cag agt gga gta cag gtt gat gcc aat 865
Arg Gly Lys Ser Met Gly Ile Gln Ser Gly Val Gln Val Asp Ala Asn
270                 275                 280                 285

tgt gaa gga gat tgc tat cac agt gga ggg aca ata ata agt aat ttg 913
Cys Glu Gly Asp Cys Tyr His Ser Gly Gly Thr Ile Ile Ser Asn Leu
                290                 295                 300

ccc ttt cag aac ata aat agc agg gca gta ggg aaa tgt ccg aga tat 961
Pro Phe Gln Asn Ile Asn Ser Arg Ala Val Gly Lys Cys Pro Arg Tyr
            305                 310                 315

gtt aag caa gag agt ctg ctg ctg gca aca ggg atg aag aat gtt ccc 1009
Val Lys Gln Glu Ser Leu Leu Leu Ala Thr Gly Met Lys Asn Val Pro
        320                 325                 330

gaa att cca aaa gga tcg cgt gtg agg aga ggc cta ttt ggt gct ata 1057
Glu Ile Pro Lys Gly Ser Arg Val Arg Arg Gly Leu Phe Gly Ala Ile
    335                 340                 345

gcg ggt ttc att gaa aat gga tgg gaa ggt ctg att gat ggg tgg tat 1105
Ala Gly Phe Ile Glu Asn Gly Trp Glu Gly Leu Ile Asp Gly Trp Tyr
350                 355                 360                 365

ggc ttc agg cat caa aat gca caa gga gag gga act gct gca gat tac 1153
Gly Phe Arg His Gln Asn Ala Gln Gly Glu Gly Thr Ala Ala Asp Tyr
                370                 375                 380

aaa agc acc caa tca gca att gat caa gta aca gga aaa ttg aac cgg 1201
Lys Ser Thr Gln Ser Ala Ile Asp Gln Val Thr Gly Lys Leu Asn Arg
            385                 390                 395

ctt ata gaa aaa act aac caa caa ttt gag tta ata gac aat gaa ttc 1249
Leu Ile Glu Lys Thr Asn Gln Gln Phe Glu Leu Ile Asp Asn Glu Phe
        400                 405                 410

act gag gtt gaa aag caa att ggc aat gtg ata aat tgg acc aga gat 1297
Thr Glu Val Glu Lys Gln Ile Gly Asn Val Ile Asn Trp Thr Arg Asp
    415                 420                 425

tcc atg aca gaa gtg tgg tcc tat aac gct gaa ctc ttg gta gca atg 1345
Ser Met Thr Glu Val Trp Ser Tyr Asn Ala Glu Leu Leu Val Ala Met
430                 435                 440                 445

gag aac cag cat aca att gat ctg acc gac tca gaa atg aac aaa cta 1393
Glu Asn Gln His Thr Ile Asp Leu Thr Asp Ser Glu Met Asn Lys Leu
                450                 455                 460

tac gaa cga gtg aag aga cta ctg aga gag aat gct gaa gaa gat ggc 1441
Tyr Glu Arg Val Lys Arg Leu Leu Arg Glu Asn Ala Glu Glu Asp Gly
            465                 470                 475

act ggt tgc ttc gaa ata ttt cac aag tgt gat gac gat tgt atg gcc 1489
Thr Gly Cys Phe Glu Ile Phe His Lys Cys Asp Asp Asp Cys Met Ala
        480                 485                 490

agt att aga aac aac aca tat gat cac agc aag tac agg gaa gag gca 1537
Ser Ile Arg Asn Asn Thr Tyr Asp His Ser Lys Tyr Arg Glu Glu Ala
    495                 500                 505

atg caa aat aga ata cag att gac cca gtc aaa cta agc agc ggc tac 1585
Met Gln Asn Arg Ile Gln Ile Asp Pro Val Lys Leu Ser Ser Gly Tyr
510                 515                 520                 525

aaa gat gtg ata ctt tgg ttt agc ttc ggg gca tca tgt ttc ata ctt 1633
Lys Asp Val Ile Leu Trp Phe Ser Phe Gly Ala Ser Cys Phe Ile Leu
                530                 535                 540

ctg gcc att gca atg ggc ctt gtc ttc ata tgt gtg aga aat gga aac 1681
Leu Ala Ile Ala Met Gly Leu Val Phe Ile Cys Val Arg Asn Gly Asn
            545                 550                 555

atg cgg tgc act att tgt ata taa gtttgg 1711
Met Arg Cys Thr Ile Cys Ile
        560

<210> 2
<211> 564
<212> PRT
<213> Avian influenza virus

<400> 2

Met Asn Thr Gln Ile Leu Val Phe Ala Leu Val Ala Ile Ile Pro Thr
1               5                   10                  15

Ser Ala Asp Lys Ile Cys Leu Gly His His Ala Val Ser Asn Gly Thr
            20                  25                  30

Lys Val Asn Thr Leu Thr Glu Arg Gly Val Glu Val Val Asn Ala Thr
        35                  40                  45

Glu Thr Val Glu Arg Thr Asn Val Pro Arg Ile Cys Ser Lys Gly Lys
    50                  55                  60

Arg Thr Val Asp Leu Gly Gln Cys Gly Leu Leu Gly Thr Ile Thr Gly
65                  70                  75                  80

Pro Pro Gln Cys Asp Gln Phe Leu Glu Phe Ser Ala Asp Leu Ile Ile
                85                  90                  95

Glu Arg Arg Glu Gly Ser Asp Val Cys Tyr Pro Gly Lys Phe Val Asn
            100                 105                 110

Glu Glu Ala Leu Arg Gln Ile Leu Arg Glu Ser Gly Gly Ile Asp Lys
        115                 120                 125

Glu Ala Met Gly Phe Thr Tyr Ser Gly Ile Arg Thr Asn Gly Thr Thr
    130                 135                 140

Ser Thr Cys Arg Arg Ser Gly Ser Ser Phe Tyr Ala Glu Met Lys Trp
145                 150                 155                 160

Leu Leu Ser Asn Thr Asp Asn Ala Ala Phe Pro Gln Met Thr Lys Ser
                165                 170                 175

Tyr Lys Asn Thr Arg Lys Asp Pro Ala Leu Ile Ile Trp Gly Ile His
            180                 185                 190

His Ser Gly Ser Thr Thr Glu Gln Thr Lys Leu Tyr Gly Ser Gly Asn
        195                 200                 205

Lys Leu Ile Thr Val Gly Ser Ser Asn Tyr Gln Gln Ser Phe Val Pro
    210                 215                 220

Ser Pro Gly Glu Arg Pro Gln Val Asn Gly Gln Ser Gly Arg Ile Asp
225                 230                 235                 240

Phe His Trp Leu Met Leu Asn Pro Asn Asp Thr Val Thr Phe Ser Phe
                245                 250                 255

Asn Gly Ala Phe Ile Ala Pro Asp Arg Ala Ser Phe Leu Arg Gly Lys
            260                 265                 270

Ser Met Gly Ile Gln Ser Gly Val Gln Val Asp Ala Asn Cys Glu Gly
        275                 280                 285

Asp Cys Tyr His Ser Gly Gly Thr Ile Ile Ser Asn Leu Pro Phe Gln
    290                 295                 300

Asn Ile Asn Ser Arg Ala Val Gly Lys Cys Pro Arg Tyr Val Lys Gln
305                 310                 315                 320

Glu Ser Leu Leu Leu Ala Thr Gly Met Lys Asn Val Pro Glu Ile Pro
                325                 330                 335

Lys Gly Ser Arg Val Arg Arg Gly Leu Phe Gly Ala Ile Ala Gly Phe
            340                 345                 350

Ile Glu Asn Gly Trp Glu Gly Leu Ile Asp Gly Trp Tyr Gly Phe Arg
        355                 360                 365

His Gln Asn Ala Gln Gly Glu Gly Thr Ala Ala Asp Tyr Lys Ser Thr
    370                 375                 380

Gln Ser Ala Ile Asp Gln Val Thr Gly Lys Leu Asn Arg Leu Ile Glu
385                 390                 395                 400

Lys Thr Asn Gln Gln Phe Glu Leu Ile Asp Asn Glu Phe Thr Glu Val
                405                 410                 415

Glu Lys Gln Ile Gly Asn Val Ile Asn Trp Thr Arg Asp Ser Met Thr
            420                 425                 430

Glu Val Trp Ser Tyr Asn Ala Glu Leu Leu Val Ala Met Glu Asn Gln
        435                 440                 445

His Thr Ile Asp Leu Thr Asp Ser Glu Met Asn Lys Leu Tyr Glu Arg
    450                 455                 460

Val Lys Arg Leu Leu Arg Glu Asn Ala Glu Glu Asp Gly Thr Gly Cys
465                 470                 475                 480

Phe Glu Ile Phe His Lys Cys Asp Asp Asp Cys Met Ala Ser Ile Arg
                485                 490                 495

Asn Asn Thr Tyr Asp His Ser Lys Tyr Arg Glu Glu Ala Met Gln Asn
            500                 505                 510

Arg Ile Gln Ile Asp Pro Val Lys Leu Ser Ser Gly Tyr Lys Asp Val
        515                 520                 525

Ile Leu Trp Phe Ser Phe Gly Ala Ser Cys Phe Ile Leu Leu Ala Ile
    530                 535                 540

Ala Met Gly Leu Val Phe Ile Cys Val Arg Asn Gly Asn Met Arg Cys
545                 550                 555                 560

Thr Ile Cys Ile
            564

## Description

The present invention is concerned with a vaccine for the protection of poultry caused by an avian pathogen comprising an attenuated infectious laryngotracheitis virus (ILTV) mutant and a pharmaceutically acceptable carrier or diluent, a cell culture infected with an attenuated ILTV mutant as well as a process for the preparation of such a vaccine.

Infectious laryngotracheitis (ILT) is a respiratory disease that mainly affects chickens, but pheasants and peacocks can also be infected. In the acute phase of the disease, from 2 to 8 days post-infection, signs of respiratory distress accompanied by gasping and expectoration of bloody exudate are observed. In addition, the mucous membranes of the trachea become swollen and hemorrhagic. This epizootic form of the disease spreads rapidly and can affect up to 100% of an infected flock. Mortality can range from 10 to 80% of the flock. Milder forms of the disease are characterized by watery eyes, conjunctivitis, persistent nasal discharge and a reduction in egg production. Also weight loss, drop in egg production and increased sensitivity to secondary infection are major causes of economic losses.
In the absence of the acute signs of the disease laboratory confirmation must be obtained. Virus can be readily isolated from tracheal or lung tissue and the demonstration of intranuclear inclusion bodies in tracheal or conjunctival tissue is diagnostic of infectious laryngotracheitis virus. In addition, rapid identification can be made with the use of fluorescent antibodies.

The etiological agent of ILT is an infectious laryngotracheitis virus (ILTV), an Alpha-herpesvirus. Apart from management adjustments, vaccination is employed as way of prevention and control, for chickens of all ages and types (parent flocks, layers, or breeders). Current vaccination strategies rely on life-attenuated vaccines that are applied preferentially via eye-drop (oculo-nasal) route. However, the presently available commercial modified live vaccines have several disadvantages. Because of the remaining virulence, they are not completely safe to apply by mass-vaccination routes; for instance, aerosol vaccination causes much vaccination reaction (in up to 10 % of the animals) and gives rise to secondary infections. Furthermore, because the presently used live vaccines are attenuated by means of serial passages in cell culture, uncontrolled mutations are introduced into the viral genome, resulting in a population of virus particles heterogeneous in their virulence and immunizing properties. In addition it has been reported that such traditional attenuated live virus vaccines can revert to virulence resulting in disease of the inoculated animals and the possible spread of the pathogen to other animals. Moreover, vaccination with existing ILTV vaccine strains results in a sero-conversion of these animals such that they can no longer be differentiated from (latent) carriers infected with more virulent field strains of ILTV.

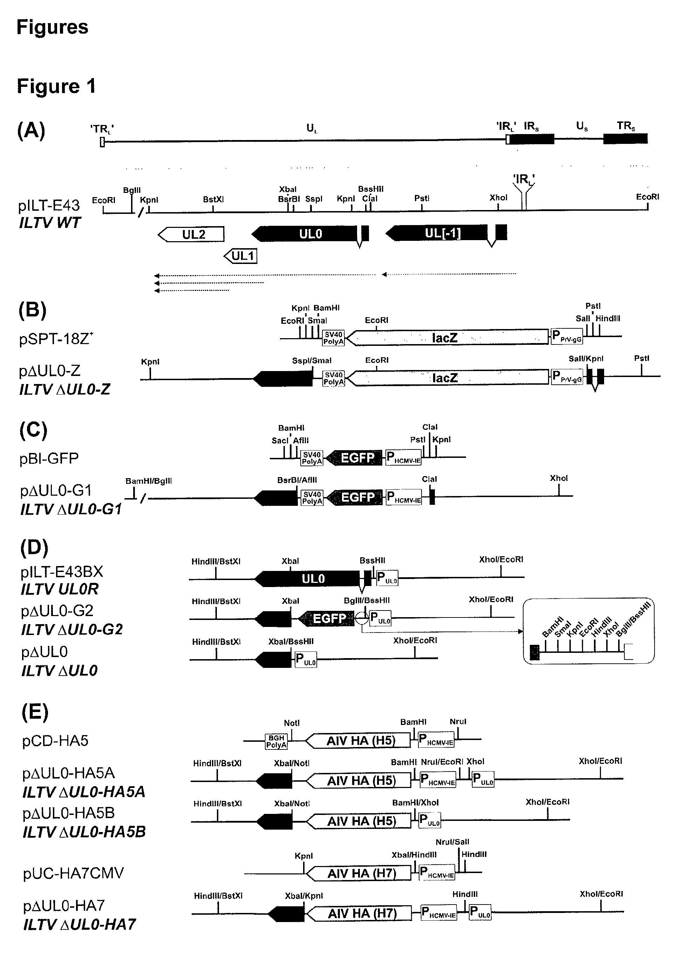

ILTV is classified as a member of the Alphaherpesvirinae subfamily of the Herpesviridae. ILTV possesses a herpesvirus type D genome consisting of a long (UL) and short (US) unique region, the latter being flanked by inverted repeat sequences (IR, TR; Figure 1). During the last years, the DNA sequence of almost the complete ILTV genome containing a linear double-stranded DNA molecule of approximately 150 kb, has been determined. Wild et al. (Virus Genes 12, 107-116, 1996) disclose the nucleotide sequence, a genomic map and organization of genes of the US region of the ILTV genome, including that of several genes encoding glycoproteins, such as gD, gE, gI, gG and gp60. Subsequently, also similar information with regard to the UL region was published by various research-groups (Fuchs and Mettenleiter, J. Gen. Virol. 77, 2221-2229, 1996 and 80, 2173-2182, 1999; Johnson et al., Arch. Virol. 142, 1903-1910, 1997).

Many of the identified ILTV genes were shown to be conserved and found in colinear arrangement compared to the herpes simplex virus (HSV) genome. Identified ILTV genes include HSV homologues, such as UL1 (gL) to UL5, UL6-UL20 and UL29 to UL42. However, despite many similarities between several parts of the ILTV- and other herpesvirus genomes, gene content and -arrangement in other parts of the genomes differ considerably. These observations, as well as phylogenetic analyses of conserved protein coding regions indicate that ILTV is only distantly related to the other herpesviruses (Ziemann et al., J. Virology 72, 6867-6874, 1998). Moreover, in contrast to other Alphaherpesviruses, ILTV exhibits both in vivo and in vitro, a very narrow host range which is restricted almost exclusively to chicken cells (Bagust et al., *In*: Diseases of Poultry, 10^{th} ed., Iowa State University Press, Ames, US, 527-539, 1997). It is anticipated that most of the ILTV-specific genomic features developed in the process of the molecular evolution of this virus to enable survival in the very specialized niche of the upper trachea of chickens.
The two recently identified, ILTV-specific, genes UL0 and UL[-1] may play a role in these unique features of ILTV. No UL0 or UL[-1] homologous genes have been found in other herpesviruses. These adjacent genes are closely related with respect to expression kinetics, mRNA structures and subcellular localization of the proteins, and display a significant amino acid sequence homology, suggesting a duplication of one ancestral gene (Ziemann et al., 1998, supra).

A prerequisite for the development of a genetically engineered, attenuated ILTV mutant vaccine is the identification of a region in the ILTV genome that is non-essential for virus infection or replication and encodes a protein that is involved in virulence of the virus. Furthermore, it is essential that the elimination of the expression of this protein does not compromise the replication of the virus mutant such that it is not able to induce a protective immune response in a vaccinated animal.

Several non-essential ILTV genes have been disclosed in the prior art. Deletion of the UL50 gene has no significant effect on ILTV replication in cell culture, however, the resulting ILTV deletion mutant displays the same pathogenicity when compared to wild-type ILTV (Fuchs et al., J. Gen. Virol. 81, 627-638, 2000 and International Herpesvirus Workshop, Boston 1999, 13.033). ILTV mutants possessing deletions of UL10 or UL49.5, encoding two envelope proteins, are viable in cell culture; however, significant growth defects (reduction of virus titer >90%) of these mutants indicate important functions of the proteins (Fuchs et al., Abstr. 2.45, 25th Int. Herpesvirus Workshop, Portland, USA, 2000). Similar growth defects have been observed in ILTV mutants having a deletion in the glycoprotein gG gene, ORF A or ORF D (Annual Virology Meeting, Vienna, April, 2000, Abstr. 6P50). In addition, Keeler and Rosenberger (US Poultry & Egg Association, Research project #253, November 1999) were not able to isolate ILTV mutants that did not express the proteins encoded by the non-essential US2 or gX gene.
Moreover, the present inventors were not able to generate ILTV mutants having a deletion in the UL[-1] gene, indicating that this ILTV-specific gene is not dispensable for ILTV infection or replication. Additionally, another ILTV-specific open reading frame (ORF A) was found to be essential for the virus (Vienna Meeting, April, 2000, Abstr. 6P50, supra).

It is an object of the present invention to provide a vaccine that comprises an ILTV vaccine strain that is attenuated in a controlled way by means of genetic engineering techniques that prevent a reversion to virulence of the attenuated vaccine strain, and that is able to induce a protective immune response in a host animal infected with the vaccine strain.

It is another object of the invention to provide a vaccine that is not only able to induce protection against ILT but also against disease caused by other avian pathogens.

These objects have been met by the present inventors by providing a vaccine for the protection of poultry against disease caused by an avian pathogen comprising an attenuated infectious laryngotracheitis virus (ILTV) mutant and a pharmaceutically acceptable carrier or diluent, characterized in that the ILTV mutant is not able to express a native UL0 protein in an infected host cell as a result of a mutation in the UL0 gene.

The inventors have found that, in contrast to the UL[-1] gene, the ILTV-specific UL0 gene is not only non-essential for ILTV infection or replication in cells but that, in addition, the inactivation of the expression of the native UL0 protein by means of controlled genetic engineering of the UL0 gene results in an ILTV mutant that is attenuated when compared to wild-type parent ILTV. Furthermore, it is found that this attenuated ILTV mutant is able to induce a protective immune response that reduces mortality and clinical signs in vaccinated animals upon challenge with virulent ILTV. In addition, the vaccine according to the present invention displays a further advantage in that it can be administered safely to chickens via spray mass-vaccination.

The localization of the ILTV-specific UL0 gene and its molecular structure is disclosed in the prior art (Ziemann et al., 1998, supra). The UL0 gene is defined herein as the open reading frame (ORF) and its promoter region upstream and partly overlapping the conserved UL1 ORF (encoding glycoprotein L) and downstream of the ILTV-specific UL[-1] ORF at the very right end of the UL genome region at the junction with the IRs sequences, located within the EcoRI fragment B (see Figure 1A).

Preferably, a vaccine according to the present invention comprises an attenuated ILTV mutant as defined above that comprises a mutation in the UL0 ORF.

An ILTV UL0 gene encodes a UL0 protein of about 506 amino acids and comprises an intron close to the 5'-end. The UL0 protein expressed from the UL0 gene in infected cells has a molecular mass of about 63 kDa and is predominantly localized in the nuclei of virus-infected cells.
With reference to the published UL0 sequence (Ziemann et al.,1998, supra) the ORF starts at nucleotide position 7152 and ends at position 5554. The UL0 promoter region spans the nucleotides 7350-7151.

ILTV strains are mainly conserved at the nucleotide level. Thus, it will be understood that for the DNA sequence of the ILTV UL0 gene natural variations can exist between individual strains within the ILTV population and that the parent virus from which the present ILTV mutant is derived can be any ILTV strain. The variation among strains may result in a change of one or more nucleotides in the UL0 gene. Typically, a UL0 gene has a nucleotide sequence encoding a protein with an amino acid sequence displaying a homology of at least 90% with the known UL0 amino acid sequence (GenBank accession no. X97256). The level of amino acid homology between two proteins can be determined with the computer program "Blast 2 Sequences", subprogram "BLASTP" that can i.a. be found at
www.ncbi.nlm.nih.gov/blast/bl2seq/bl2.html. Reference for this program is further made to Tatusova and Madden, FEMS Microbiol. Letters 174, 247-250, 1999. The matrix used is "blosum62" and the parameters are the default parameters: open gap: 11, extension gap: 1, Gap x_ deopoff: 50.
It is clear that a vaccine based on an ILTV mutant derived from such ILTV strains are also included within the scope of the invention.
Preferably, a vaccine according to the invention is based on an ILTV mutant that comprises a mutation in the UL0 gene having a nucleotide sequence encoding a UL0 protein having an amino acid sequence published in; GenBank accession no. X97256).

A mutation is understood to be a change of the genetic information in a wild type or unmodified UL0 gene of a parent ILTV strain that is able to express a native UL0 protein. The mutation attenuates the virus, rendering it suitable for use as a vaccine strain against ILT.
The mutation can be an insertion, deletion and/or substitution of one or more nucleotides in the UL0 gene.

To prevent adverse effects of a mutation in the 3'-end of the UL0 ORF that overlaps with the UL1 gene on the forming of viable recombinant ILTV UL0 mutants, with the term "a mutation in the UL0 gene" is meant a mutation in the UL0 gene in a region that does not overlap with the UL1 promoter region and ORF. With reference to the published UL0 and UL1 sequences (GenBank accession no. X97256) the UL1 promoter region starts at about nucleotide 5900 and the UL1 ORF starts at position 5570.

With the term "is not able to express a native UL0 protein" is meant that the ILTV vaccine strain used herein expresses a protein in an infected host cell that can be distinguished by conventional tests from the 63 kDa UL0 protein expressed by a wild-type ILTV, or does not express a UL0 protein at all. For example, in the former case the ILTV mutant expresses only a fragment of the wild-type UL0 protein.
Preferably, the ILTV mutant vaccine strain used herein expresses no UL0 protein upon infection and replication in a host cell.
To assay an ILTV mutant for the expression of the native UL0 protein by a serological test, first, mono-specific UL0 antiserum is generated. For this purpose the UL0 ORF or parts thereof can be expressed as fusion protein in E. coli. The fusion protein is purified by affinity chromatography or gel-electrophoresis and the purified preparation is used to immunize rabbits for the production of the antiserum (Ziemann et al., 1998, supra). Second, viruses are grown in a cell culture, harvested, lysed and immunoprecipitated, if desired. The proteins are separated in polyacrylamide gels and transferred to nitrocellulose using well-known procedures. Subsequently, the gels are incubated with the antiserum raised against the fusion protein and the presence or absence of a native 63 kDa protein can be determined.
In a similar assay, the presence or absence of expressed native UL0 can be determined by radioactive labeling of the ILTV proteins during culturing and immunoprecipitating the viral harvest with anti-UL0 antiserum (Ziemann et al., 1998, supra).

A typical ILTV substitution mutant to be used in the present invention comprises a substitution of one or more nucleotides that result in the changes of one or more codons in the ORF into a stop codon, preferably in the 5'-half of the ORF. Alternatively, the substitution may result in a change and removal of the start codon of the UL0 ORF.

In a preferred aspect of this embodiment the vaccine according to the present invention comprises a deletion in the UL0 gene. The deletion disrupts the expression of the native UL0 protein and can range from one nucleotide to almost the complete ORF with the exception of the part that overlaps with the UL1 gene. Particular effective deletions are those that are made in the 5'-half of the UL0 gene and/or that result in a shift of the reading frame.
In particular, the deletions introduced into the ILTV vaccine strain described above comprise at least 10 nucleotides, more preferably at least 100 nucleotides, most preferred at least 500 nucleotides.
A particularly useful ILTV deletion mutant contains a deletion of a 546 bp KpnI/SspI-fragment encoding aa 49-231, a 984 bp ClaI/BsrBI-fragment encoding aa 17-318 or a 1137 bp BssHII/XbaI-fragment encoding aa 1-352.

A useful ILTV mutant as defined above can also be obtained by the insertion of a heterologous nucleic acid sequence into the UL0 gene, i.e. a nucleic acid sequence that is different from a nucleic acid sequence naturally present at that position of the ILTV genome. Preferably, the heterologous nucleic acid sequence is a DNA fragment not present in the ILTV genome. The heterologous nucleic acid sequence can be derived from any source, e.g. synthetic, viral, prokaryotic or eukaryotic.
Such a nucleic acid sequence can inter alia be an oligonucleotide, for example of about 10-60 bp, if desired also containing one or more translational stop codons (see US patent 5,279,965), or a polynucleotide encoding a polypeptide.

In a further aspect of this embodiment a vaccine according to the present invention comprises an ILTV deletion that contains a heterologous nucleic acid sequence in place of the deleted ILTV DNA.

An ILTV mutant as described above comprising a heterologous nucleic acid sequence can also be used as a vector for delivering a heterologous polypeptide in poultry.
Therefore, the present invention also provides a vaccine comprising an ILTV mutant as described above wherein the heterologous nucleic acid sequence encodes an antigen of an avian, in particular a chicken, pathogen, that can be used not only for the protection of poultry against ILT but also against disease caused by other avian pathogens. Such a vector vaccine that is based on a live attenuated ILTV is able to immunize chickens against other pathogens by the replication of the ILTV mutant in the vaccinated host animal and the expression of the foreign antigen that triggers an immune response in the vaccinated animal.

Preferably, the ILTV vector mutant comprises a heterologous nucleic acid sequence encoding a protective antigen of avian influenza virus (AIV), Marek's disease virus (MDV), Newcastle disease virus (NDV), infectious bronchitis virus (IBV), infectious bursal disease virus (IBDV), chicken anemia virus, reo virus, avian retro virus, fowl adeno virus, turkey rhinotracheitis virus (TRTV), E. coli, Eimeria species, Cryptosporidia, Mycoplasms, such as M. gallinarum, M. synoviae and M. meleagridis, Salmonella-, Campylobacter-, Ornithobacterium (ORT) and Pasteurella spp.
More preferably, the ILTV vector mutant comprises a heterologous nucleic acid sequence encoding an antigen of AIV, MDV, NDV, IBV, IBDV, TRTV, E. coli, ORT and Mycoplasma

In particular, the ILTV vector mutant may comprise a hemagglutinin (HA) gene of AIV (Flexner et al., Nature 335, 259-262, 1988; GenBank Accession No. AJ305306), the gA, gB or gD gene of MDV (Ross et al., J. Gen. Virol. 74, 371-377, 1993; WO 90/02803), the HN or F gene of NDV (Sondermeijer et al., Vaccine 11, 349-358, 1993) or the VP2 gene of IBDV (Bayliss et al., Arch. Virol. 120, 193-205,1991).
In an even more preferred embodiment a vaccine as described above is provided that is based on an attenuated ILTV mutant comprising an HA gene of AIV.

In particular, a vaccine is contemplated that is based on the attenuated ILTV mutant comprising an H5 or H7 hemagglutinin gene of AIV.

Alternatively, the ILTV vector mutant comprises a heterologous nucleic acid sequence encoding an immuno-modulator such as an (avian) interferon, cytokine or lymphokine. An immuno-modulator expressed by the ILTV mutant enhances the immune response induced by the ILTV mutant and as such contributes to an enhanced protection. Therefore, the present invention also provides a vaccine comprising an ILTV mutant as described above that contains a heterologous nucleic acid sequence encoding an immuno-modulator.

An essential requirement for the expression of the heterologous nucleic acid sequence by an ILTV mutant as described above is an adequate expression control sequence, particularly a promoter and a poly-adenylation signal, operably linked to the heterologous nucleic acid sequence. Such expression control sequences are well known in the art, in particular for the construction of herpesvirus vectors, and extend to any eukaryotic, prokaryotic or viral promoter or poly-A signal capable of directing gene transcription in cells infected by the ILTV mutant. Examples of useful promoters are the SV-40 promoter (Science 222, 524-527, 1983), the metallothionein promoter (Nature 296, 39-42, 1982), the heat shock promoter (Voellmy et al., Proc. Natl. Acad. Sci. USA 82, 4949-53, 1985), the PRV gX promoter (Mettenleiter and Rauh, J. Virol. Methods 30, 55-66, 1990), the human cytomegalovirus IE promoter (US patent no. 5,168,062), the Rous Sarcoma virus LTR promoter (Gorman et al., PNAS 79, 6777-6781, 1982), human elongation factor 1α or ubiquitin promoter, or promoters present in ILTV, in particular the UL0 promoter. Examples of useful poly-A signals are the rabbit β-globin-, the SV40- and the bovine growth hormone poly-A signal. Alternatively, the endogenous poly-A signals of UL0, UL1 or UL2 can be used.

Therefore, a preferred vaccine according to the invention is based on an ILTV mutant that comprises a heterologous nucleic acid sequence encoding a polypeptide as described above that is under the control of an expression control sequence.

In still a further aspect of the present invention a vaccine is provided comprising an ILTV mutant as described above that additionally comprises a further attenuating mutation in the ILTV genome. For example, such a vaccine is based on a modified live vaccine strain, like those presently commercially available (e.g. Nobilis ILT®, BioTrach®, Trachine®) or on a genetically engineered ILTV that fails to express an additional protein involved in virulence, such as gE, gl, gM, TK, RR, UL21, UL50 or PK (Schnitzlein et al., Virology 209, 304-314, 1995; Mettenleiter, Abstracts from ESW meeting, 27-30 August, 2000, 15-17; WO 96/29396).

The well-known procedures for inserting DNA sequences into cloning/expression vectors and in vivo homologous recombination can be used to introduce a mutation into the ILTV genome.

In principle, this can be accomplished by constructing a recombinant transfer vector for recombination with genomic ILTV DNA that comprises a vector capable of replication in a host cell and a relevant ILTV DNA fragment harboring the desired mutation. Such a recombinant transfer vector may be derived from any suitable vector known in the art for this purpose, such as a plasmid, cosmid, virus or phage, a plasmid being most preferred. Examples of suitable cloning vectors are plasmid vectors such as pBR322, the various pUC, pEMBL and Bluescript plasmids, bacteriophages, e.g. lambda, charon 28 and the M13mp phages.
Suitable transfer vectors, host cells and methods of transformation, culturing, amplification, screening etc. can be selected by one skilled in the art from the well known options in this field (see for example, Rodriguez, R.L. and D.T. Denhardt, edit., Vectors: A survey of molecular cloning vectors and their uses, Butterworths, 1988; Current Protocols in Molecular Biology, eds.: F.M. Ausubel et al., Wiley N.Y., 1995; Molecular cloning: a laboratory manual, 3^{rd} ed.; eds: Sambrook et al., CSHL press, 2001 and DNA Cloning, Vol. 1-4, 2^{nd} edition 1995, eds.: Glover and Hames, Oxford University Press).

Briefly, first, an ILTV DNA fragment comprising UL0 nucleic acid sequences is inserted into a transfer vector using standard recDNA techniques. The ILTV DNA fragment may comprise part of the UL0 ORF or the complete UL0 ORF, and if desired flanking sequences thereof.
Second, if an ILTV UL0 deletion mutant is to be obtained part of the UL0 ORF is deleted from the recombinant transfer vector. This can be achieved for example by appropriate exonuclease III digestion or restriction enzyme cleavage of the recombinant vector insert or via careful selection of PCR primers. In case an ILTV insertion mutant is to be obtained a heterologous nucleic acid sequence, and if desired a DNA fragment comprising expression control sequences, are inserted into the UL0 nucleic acid sequences present in the recombinant vector or in place of deleted UL0 nucleic acid sequences. The ILTV DNA sequences that flank the mutation introduced in the ILTV DNA should be of appropriate length as to allow homologous recombination with genomic ILTV DNA to occur. Generally, flanking sequences of 500 bp or larger allow efficient homologous recombination.

Thereafter, cells, for example chicken embryo liver cells, chicken kidney cells, or preferably, the chicken hepatoma cell line LMH (Schnitzlein et al., Avian Diseases 38, 211-217, 1994) are co-transfected with ILTV genomic DNA in the presence of the recombinant transfer vector containing the mutated ILTV DNA insert whereby recombination occurs between this insert and the ILTV genome.
In a particularly advantageous process for the construction of the recombinant ILTV mutant, the recombinant transfer vector containing the mutated ILTV DNA insert and ILTV genomic DNA are used for (calcium-phosphate mediated) co-transfection of LMH cells in the presence of an expression vector (e.g. pRc-UL48) encoding the ILTV homologue of the herpesviral trans-activator αTIF (UL48) and/or the regulatory protein ICP4, because both increase the infectivity of naked ILTV DNA (Fuchs et al., J. Gen. Virol. 81, 627-638, 2000).

Recombinant viral progeny is thereafter produced in cell culture and can be selected genotypically or phenotypically. For example, by hybridization or by detecting the presence or absence of enzyme activity or another screenable marker, such as green fluorescent protein, or β-galactosidase encoded by a gene inserted or removed during the preparation of the recombinant transfer vector.
Transfection progenies are analyzed by plaque-assays and the plaques displaying the expected genotype or phenotype are picked by aspiration. Subsequently, an ILTV mutant as described above can be purified to homogeneity by limiting dilutions on (chicken embryo kidney) cells grown in microtitre plates.

A vaccine according to the invention can be prepared by conventional methods such as for example commonly used for the commercially available live- and inactivated ILTV vaccines. Briefly, a susceptible substrate is inoculated with an ILTV mutant as described above and propagated until the virus replicated to a desired infectious titre after which ILTV containing material is harvested.

Every substrate which is able to support the replication of ILT viruses can be used in the present invention, including primary (avian) cell cultures, such as chicken embryo liver cells (CEL) or chicken embryo kidney cells (CEK) or an avian cell line, such as LMH. Usually, after inoculation of the cells, the virus is propagated for 3-10 days, after which the infected cells and/or the cell culture supernatant is harvested. The infected cells can be freeze-thawed to free the virus followed by storage of the material as frozen stock.

Alternatively, the ILTV mutant can be propagated in embryonated SPF chicken eggs. Embryonated eggs can be inoculated with, for example 0.2 ml ILTV mutant containing suspension or homogenate comprising at least 10¹ TCID₅₀ per egg, and subsequently incubated at 37 °C. After about 2-6 days the ILT virus product can be harvested by collecting the embryo's and/or the membranes and/or the allantoic fluid followed by appropriate homogenizing of this material.

A live vaccine according to the invention contains an ILTV mutant as described above and a pharmaceutically acceptable carrier or diluent customary used for such compositions. The vaccine can be prepared and marketed in the form of a suspension or in a lyophilised form. Carriers include stabilisers, preservatives and buffers. Suitable stabilisers are, for example SPGA, carbohydrates (such as sorbitol, mannitol, starch, sucrose, dextran, or glucose), proteins (such as dried milk serum, albumin or casein) or degradation products thereof. Suitable buffers are for example alkali metal phosphates. Suitable preservatives are thimerosal, merthiolate, gentamicin and neomycine. Diluents include sterilized physiological saline, aqueous phosphate buffer, alcohols and polyols (such as glycerol).

If desired, the live vaccines according to the invention may contain an adjuvant.

Although administration by injection, e.g. intramuscularly, or subcutaneously of the live vaccine according to the present invention is possible, the vaccine is preferably administered by the inexpensive mass application techniques commonly used for poultry vaccination. For ILTV vaccination these techniques include drinking water and aerosol- or spray vaccination.

A preferred method for the administration of a vaccine according to the invention is by coarse spray using nozzle droplet sizes of > 100µm, particularly in the presence of much diluent, at > 250 ml per 1000 animals. Appropriate spraying is directed at the eyes and mouth of the animals. This will mimic the oculo-oro-nasal routes of vaccination and induce the desired immunization.

Alternative methods for the administration of the live vaccine include in ovo, eye-drop, oro-nasal- and beak dipping administration.

In another aspect of the present invention a vaccine is provided comprising an ILTV mutant in an inactivated form. A vaccine containing the inactivated ILTV mutant can, for example comprise one or more of the above-mentioned pharmaceutically acceptable carriers or diluents suited for this purpose. Preferably, an inactivated vaccine according to the invention comprises one or more compounds with adjuvant activity.

The vaccine according to the invention comprises an effective dosage of an ILTV mutant as the active component, i.e. an amount of immunising ILTV mutant as described above that will induce protection in the vaccinated birds against challenge by a virulent virus. Protection is defined herein as the induction of a significantly higher level of protection in a population of birds after vaccination compared to an unvaccinated group. Generally, protection induced by an ILTV vaccine is assayed by determining mortality and clinical signs of respiratory disease, such as described in Example 3.

Typically, the live vaccine according to the invention can be administered in a dose of 10¹-10⁷ tissue culture infectious dose 50% (TCID₅₀) per animal, preferably in a dose ranging from 10²-10⁵ TCID₅₀. An inactivated vaccine may contain the antigenic equivalent of 10³-10⁹ TCID₅₀ per animal.

Inactivated vaccines are usually administered parenterally, e.g. intramuscularly or subcutaneously.

Although, the ILTV vaccine according to the present invention may be used effectively in chickens, also other poultry may be successfully vaccinated with the (vector) vaccine. Chickens include broilers, layers and reproduction stock.

The age of the animals receiving a live or inactivated vaccine according to the invention is the same as that of the animals receiving the conventional live- or inactivated ILTV vaccines. For example, chickens may be vaccinated at four weeks of age or earlier in case of an emergency. Breeders and layers usually receive a second vaccination at 8-16 weeks of age.

The invention also includes combination vaccines comprising, in addition to the ILTV mutant, one or more vaccine antigens, such as a live or inactivated vaccine virus or bacterium, derived from other pathogens infectious to poultry.
Preferably, the combination vaccine additionally comprises one or more vaccine strains of AIV, MDV, HVT, IBV, NDV, TRTV, reovirus, E. coli, ORT, Salmonella spp, Campylobacter spp, Mycoplasma's or Eimeria spp.

### Legends to the figures

### Figure 1

Genomic map of ILTV genome and construction of the transfer plasmids. The relevant restriction sites for generation of the transfer plasmids, the heterologous sequences, promoters and poly-A signals are indicated. ILTV recombinants (names in bold italics) could be isolated after cotransfection of cells with transfer plasmids and virion-DNA.

### Figure 2

Lysates of non-infected (n.i.) and ILTV-infected (5 pfu/cell, 24 h p.i.) CEK cells were separated on discontinuous SDS-10% Polyacrylamide gels. Western blots were incubated with ULO-, or gC-specific antibodies. Binding of peroxidase-conjugated secondary antibodies was detected by chemiluminescence, and monitored on X-ray films. Molecular weight markers are indicated at the left.

### Figure 3

Graphical representation of the scores for the clinical signs of respiratory disease observed in the animal trials that were performed to determine the residual pathogenicity of the ILTV mutants ΔUL0, ΔUL0-LacZ and ΔUL0-HA7, next to appropriate controls.

Scores are averages per treatment group per day and are determined as outlined in Example 3.

### Examples

### Example 1

### Preparation of ILTV UL0 deletion and insertion mutants

### Construction of transfer plasmids for deletion of ILTV UL0 gene sequences and insertion of reporter genes.

Virus DNA was isolated from ILTV strain A489 infected primary chicken embryonic kidney (CEK) cells by lysis with N-lauroylsarcosinate, RNase- and pronase treatment, phenol extraction, and ethanol precipitation (Fuchs and Mettenleiter, J. Gen. Virol. 77: 2221-2229, 1996). After digestion with different restriction endonucleases the obtained ILTV DNA fragments were cloned into commercially available plasmid vectors. Plasmid pILT-E43 (Figure 1A) contains the 11298 bp EcoRI-fragment B of a pathogenic ILTV strain in pBS (-) (Stratagene). The cloned DNA fragment includes the unique ILTV genes UL0 and UL[-1] which were shown to be expressed from spliced mRNA's (Ziemann et al., supra, 1998).

Several reporter gene plasmids were constructed and utilized for deletion of the ILTV UL0 gene. For expression of β-galactosidase (Figure 1B), a 3.5 kbp SaII-BamHI fragment containing the E. coli LacZ gene under control of the pseudorabies virus glycoprotein G gene promoter (Mettenleiter and Rauh, J. Virol. Methods 30, 55-66, 1990) was recloned in pSPT-18 (Roche). Furthermore, the SV40 polyadenylation signal was provided by substitution of the 3'-part of the insert by a 450 bp EcoRI-BamHI fragment derived from pCH110 (Amersham-Pharmacia). The resulting vector pSPT-18Z⁺ (Figure 1B) was modified by insertion of ILTV-DNA sequences at both ends of the reporter gene. Subsequently, a 944 bp Kpnl-Pstl fragment, and a 2223 bp Kpnl-Sspl fragment were recloned from pILT-E43 into pSPT-18Z⁺ which had been doubly digested with Pstl and SaII, or Smal and Kpnl, respectively. Before ligation, non-compatible cohesive ends were blunted by treatment with Klenow polymerase. Thus, the obtained transfer plasmid pΔUL0-Z (Figure 1B) exhibits a 546 bp deletion within the UL0 open reading frame, and contains the LacZ expression cassette in parallel orientation with the affected ILTV gene.

All constructs used for expression of the enhanced green fluorescent protein (EGFP) were derived from pEGFP-N1 (Clontech). From this plasmid, the multiple cloning site located between the human cytomegalovirus immediate early gene promoter (P_{HCMVIE}), and the EGFP open reading frame was removed by double digestion with Bglll and BamHI followed by religation. To obtain pBI-GFP (Figure 1C), the modified expression cassette was excised as a 1581 bp AseI-AfIII fragment, treated with Klenow polymerase, and inserted into the polylinker region of the Smal-digested vector pBluescript SK(-) (Stratagene). The transfer plasmid pΔUL0-G1 (Figure 1C) was generated by subsequent insertion of 3003 bp BgIII-BsrBI, and 1818 bp Clal-Xhol fragments of pILT-E43 into pBI-GFP which had been doubly digested with BamHI and AfIII, or Clal and Xhol. The preformed deletion embraces 984 bp of the ILTV UL0 gene including the entire intron sequence, and the reporter gene insertion is again in parallel orientation with the deleted virus gene.

Since previous studies revealed an abundant expression of the UL0 protein in ILTV infected cells, the suitability of the UL0 gene promoter for foreign gene expression was tested. To remove undesired restriction sites, the insert of pILT-E43 was subsequently shortened by HindIII-BstXI, and XhoI-EcoRI double digestions, followed by Klenow treatment and religation. From the resulting plasmid pILT-E43BX (Figure 1D), an 1141 bp XbaI-BssHII fragment including the initiation codon of UL0 was removed, and substituted by an 802 bp XbaI-BgIII fragment of pEGFP-N1 (Clontech) which contains the EGFP open reading frame without any promoter sequences. Whereas in pΔUL0-G2 the major part of the viral UL0 reading frame was replaced by that of EGFP, the simultaneously constructed plasmid pΔUL0 exhibits the same deletion, but contains no foreign DNA sequences (Figure 1D).

### Construction of transfer plasmids and AIV HA expressing ILTV mutants

The hemagglutinin (HA) gene of the recently isolated, highly pathogenic H5N2 subtype AIV A/Italy/8/98 was reverse transcribed, cloned in the eucaryotic expression vector pcDNA3 (Invitrogen), and sequenced (Lüschow et al., Vaccine, vol. 19, p. 4249-4259, 2001, and GenBank Accession No. AJ305306). From the obtained expression plasmid pCD-HA5 the HA gene together with HCMV-IE promoter was inserted as a 2646 bp Nrul/Notl-fragment into the EcoRl/Xbal doubly-digested plasmid pΔUL0-G2 after Klenow fill-in of the single-stranded overhangs. In the resulting plasmid pΔUL0-HA5A, the EGFP reading frame has been replaced by a HA expression cassette, which is in parallel orientation with UL0 to utilize the common polyadenylation signal of UL0, UL1, and UL2. Finally, the HCMV-promoter was removed by digestion with BamHI and Xhol, Klenow-treatment, and religation. Thus, from plasmid pΔUL0-HA5B the hemagglutinin can be now expressed under control of the ILTV UL0 gene promoter.

In a second approach, the HA gene of the highly pathogenic H7N1 subtype AIV A/Italy/445/99 was reverse transcribed, and amplified by PCR. The 1711 bp product was cloned in the Smal-digested vector pUC18 (Amersham), and sequenced (seq. id. no. 1). The resulting plasmid was doubly-digested with Xbal and HindIII and, after Klenow-treatment, the HCMV-IE promoter was inserted at the 5'-end of the HA open reading frame as a 681 bp HindIII/NruI fragment of pcDNA3. Subsequently, the HA expression cassette was recloned as a 2437 bp KpnI/HindIII-fragment in theXba/HindIII doubly-digested plasmid pΔUL0-G2 after blunting of non-compatible single-stranded overhangs. The finally obtained plasmid pΔUL0-HA7 (Fig. 1E) contains the H7 type HA gene in parallel orientation with the deleted UL0 open reading frame of ILTV, but under control of the HCMV-IE promoter.

The three transfer plasmids (Figure 1E) were used for co-transfection of cells together with virus DNA of ILTV ΔUL0-G1 which facilitated selection of the desired non-fluorescent ILTV recombinants.

### Generation of recombinant ILTV UL0 mutants

Because the infectivity of isolated ILTV DNA in transfected CEK or chicken hepatoma cells is very low, expression plasmids of viral transactivators were generated. For that purpose, the UL48 open reading frame of ILTV (Ziemann et al., J. Virology 72: 847-852, 1998) encoding the putative homologue of an alphaherpesvirus transactivator (VP16, αTIF; Roizman and Sears, Fields Virology 3^{rd} edn: 2231-2295, 1996) was recloned as a 2259 bp Ncol-Spel fragment in pRc-CMV (Invitrogen), which permits constitutive gene expression under control of the HCMV-IE promoter. After calcium phosphate cotransfection of cells (Graham and van der Eb, Virology 52: 456-467, 1973) with ILTV-DNA and the expression plasmid pRc-UL48, virus plaque numbers were substantially increased when compared to results obtained with control plasmids or without any plasmid (Fuchs et al., 2000, supra).

For generation of virus recombinants, CEK or LMH cells were cotransfected with ILTV DNA, pRc-UL48, and the desired transfer plasmids. After 5 to 7 days the cells were scraped into the medium, and lysed by freezing and thawing. Virus progeny was analyzed by limiting dilutions on CEK cells grown in 96 well plates. Whereas EGFP-expressing ILTV recombinants could be identified directly by fluorescence microscopy, β-galactosidase activity was detected by *in vivo* staining with medium containing 300 µg/ml BluoGal (Gibco BRL). Virus recombinants were harvested, and purification was repeated until all plaques exhibited the expected phenotype. Finally, virus DNA was prepared and characterized by restriction analyses, Southern blot hybridization, and PCR to verify the correct deletions or insertions.

Virus DNA of a pathogenic wild type strain was used for co-transfections to obtain the ILTV recombinants ΔUL0-Z, ΔUL0-G1, and ΔUL0-G2 (Figure 1B, 1C, and 1D). For generation of a rescue mutant (ILTV UL0R; Figure 1A), a deletion mutant without foreign sequences (ILTV ΔUL0; Figure 1D), and of HA expressing recombinants (ILTV ΔUL0-HA5A, ILTV ΔUL0-HA5B, ILTV ΔUL0-HA7; Figure 1E) co-transfections were performed with DNA of ILTV ΔUL0-G1, and pILT-E43, or ΔUL0, or the respective derivatives of ΔUL0-G2. In these cases, the virus progenies were screened for non-fluorescent plaques on CEK cells.

### In vitro characterization of recombinant ILTV UL0 mutants

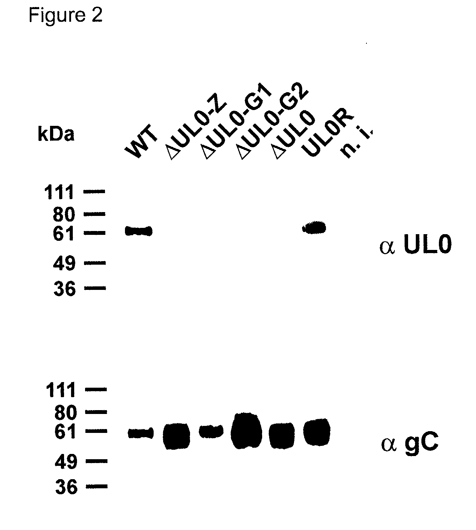

To confirm that the isolated UL-deletion mutants of ILTV do not express the native UL0 gene product, CEK cells were infected with at a m.o.i. of 5 pfu/cell with the respective deletion mutants, and incubated for 24 h. at 37 °C. Then the cells were lysed, proteins were separated on discontinuous SDS-polyacrylamide gels, and transferred to nitrocellulose filters according to standard techniques. Western blots were incubated and processed as described (Fuchs and Mettenleiter, J. Gen. Virol. 80, 2173-2182, 1999) with UL0 specific rabbit antiserum (Ziemann et al., supra, 1998) or with a monoclonal gC-specific antibody. All lanes showed reaction with the Moab, however, only in cells infected with either wild-type virus or an UL0 rescue mutant the 63 kDa UL0 protein was detectable (Figure 2). There is no evidence that any of the UL0 deletion mutants stably expressed a smaller protein from the non-deleted parts of the gene. Western blot analyses of infected cell-lysates with AIV subtype-specific chicken antisera further confirmed abundant expression of the H7 type hemagglutinin by ILTV ΔUL0-HA7, and of the H5 type hemagglutinin by ILTV ΔUL0-HA5A, whereas the foreign protein was not clearly detectable in cells infected with ILTV ΔUL0-HA5B.

### Example 2

### Culture and titration of ILTV UL0 mutants

Preparation of recombinant and control ILT viruses was performed by inoculation onto the dropped chorio-allantoic membrane (CAM) of 9 to 11 days old embryonated SPF chicken eggs, using techniques known in the art. After incubation for 5 to 6 days at 37 °C, the CAM's were harvested, homogenized, filtrated through a 100 µm filter and titrated.

Titration of the viruses in LMH cells is performed on Leghorn male hepatoma cells (LMH). In 96 well plates, semi-confluent monolayers of LMH cells are infected with stepwise dilutions of an ILT virus-sample. Appropriate positive and negative controls were included. The plates are incubated for 5 days, the cells are fixed with ice-cold ethanol, and stained for presence of ILT virus with a standard immunofluorescence protocol, using a polyclonal chicken antiserum against ILT, and an anti-chicken IgG goat antibody, coupled to FITC. Wells that show bright green fluorescence where ILT virus has replicated, are considered positive. Titers are presented as Log₁₀ TCID₅₀ values, using the Spearman-Kärber algorithm.

For a recombinant ILTV to be applicable as vaccine for mass application good growth-yields are essential, therefore the applied gene deletion should not interfere with its capacity to grow to high titers. Apart from ΔUL0 several more ILT recombinants carrying gene deletions that cause absence of the corresponding gene product have been constructed, and these have all been inoculated into fertilized eggs for producing virus from CAM homogenate. Several incubations and harvests were performed to obtain the maximal yields possible for a certain recombinant. Surprisingly the UL0 deletion allows replication in eggs to titers which are at least as good as the yields of the undeleted wild type parental virus, while the other ILTV deletion-recombinants produce much less, or undetectable virus yields. This favourable capacity is maintained when genes of LacZ or AIV H7 are inserted, see Table 1.

**Table 1:**

| The deletion recombinants tested and the maximal yield of rec. ILT virus in CAM homogenate | | | |
|---|---|---|---|
| | | | max. yield: |
| recILTV | deletion in: | insertion of: | (Log₁₀ TCID₅₀/ml) |
| ΔgG+Z: | gG (Us4) | Lac Z gene | 3.5 |
| ΔUL10+Z | UL10 (gM) | Lac Z gene | 2.7 |
| ΔUL21+Z | UL21 | Lac Z gene | 3.7 |
| ΔUL49.5+Z | UL49.5 (gN) | Lac Z gene | < 2.5 |
| ΔUL0+Z | UL0 | Lac Z gene | 5.1 |
| ΔUL0+HA7 | UL0 | AIV H7 gene | 5.9 |
| ΔUL0 | UL0 | none | 5.7 |
| ΔUL50 | UL50 | none | 4.7 |
| ΔTK | UL23 | none | 4.8 |
| ΔOrf B | Orf B | none | 4.4 |
| A489 | (wild type) | | 5.2 |

### Example 3

### Animal trials to determine the attenuation of ILTV UL0 mutants

Animal experiments were performed to assess the level of attenuation obtained by introducing deletions/insertions in the UL0 gene. The standard test for this purpose for ILTV is to inoculate a virus sample directly into the trachea of susceptible chickens, and observe the level of clinical signs, or the number of animals killed for 9 to 10 days. As comparison, virus samples (homogenized, and filtered CAM) were prepared of the virulent wild-type ILT strain A 489 that had served as donor for the viral DNA that had been mutated, and a similar sample from mock infected CAM's. It is important to test different dosages, as pathology in ILT infection is directly related to the dosage received.

Therefore virus samples were amplified and titrated on LMH cells in triplo as described above, and used for inoculation of 10-day-old SPF chicks, via the intratracheal route, at 0.2 ml per animal. The different treatment groups were housed individually in groups of 20 animals, in negative pressure isolators. The chicks were observed for 9 days, and clinical signs related to respiratory disease were scored daily, according to the following table:
score 0: no signs of (respiratory) disease
score 1: light respiratory distress; animal slow, depressed, some coughing, head shaking
score 2: serious respiratory distress; gasping, pump-breathing, coughing, animal lying down, conjunctivitis, nasal exudate.
score 3: animal dead

In experiment Path 1, ΔUL0+Z was compared to uninfected and to A 489 infected CAM. ΔUL0+Z was tested in two dosages.

In experiment Path 2, the same experiment was repeated a second time, with the same treatment protocol. This time ΔUL0 recombinants were included, which were also tested in two dosages.

Finally in experiment Path 3, a similar experiment was performed, this time 2 dosages of recombinant ILT viruses were tested that carried the AIV HA7 insert in the UL0 gene locus.

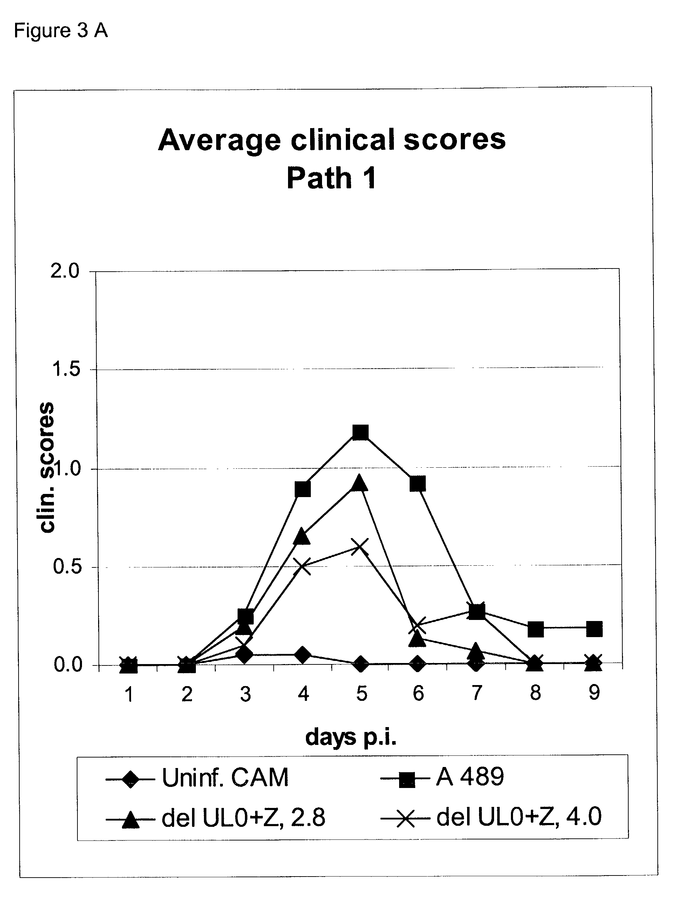

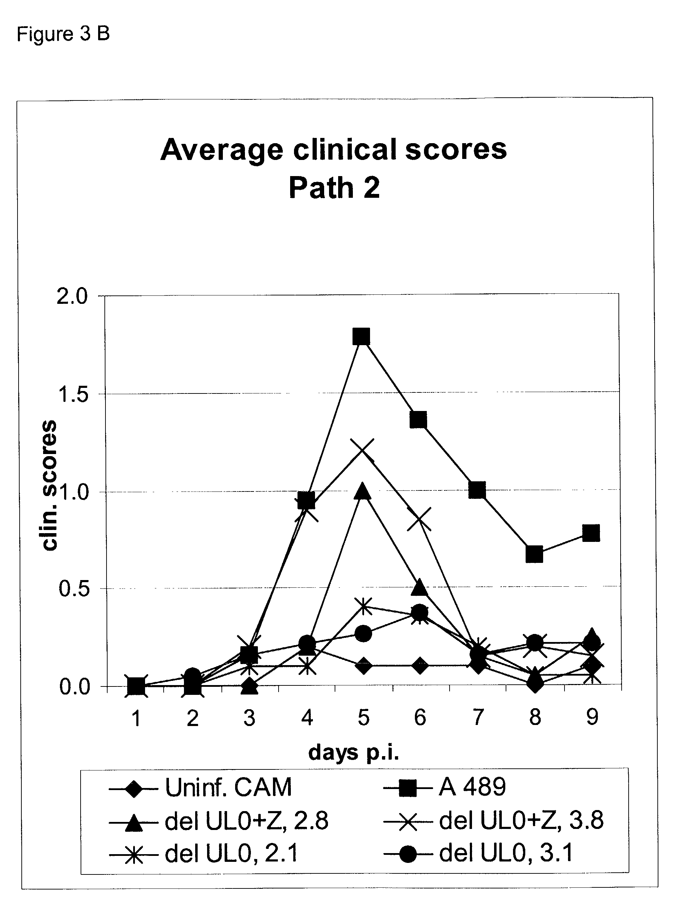

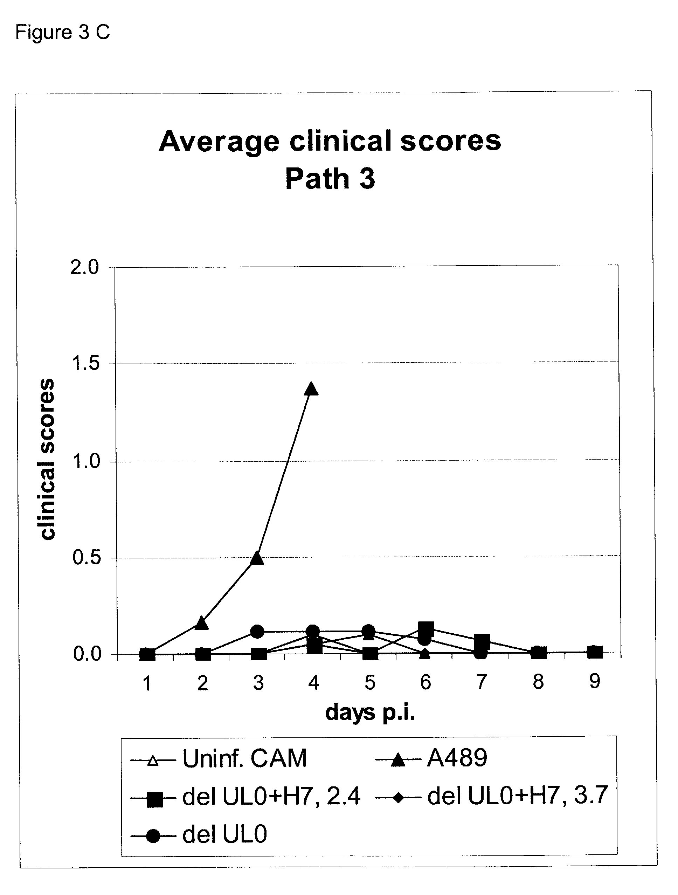

The results (presented in Table 2, and in Figure 3 A - C) show that all UL0 deletions induce considerably less mortality compared to the wild-type virus they originate from. The seriousness of clinical signs is reduced significantly; in ΔUL0+Z and ΔUL0 some residual pathogenicity remains. Insertion of the AIV H7 insert further reduces this to zero. However all three recombinants conserve the property to replicate effectively in the trachea, and upon inoculation onto CAM (see Table 1).

**Table 2:**

| Attenuation of the ILTV UL0 mutants | | | | |
|---|---|---|---|---|
| | | | Mortality | |
| | Name | Dose *) | #/total | % |
| Path 1 | Uninf. CAM | < 2.5 | 0/20 | 0 |
| | A 489 | 3.2 | 9/20 | 45 |
| | del UL0+Z | 2.8 | 1/20 | 5 |
| | | 4.0 | 0/20 | 0 |
| | | | | |
| Path 2 | Uninf. CAM | < 2.5 | 1/20 | 5 |
| | A 489 | 2.6 | 10/19 | 53 |
| | del UL0+Z | 2.8 | 0/20 | 0 |
| | | 3.8 | 0/20 | 0 |
| | del UL0 | 2.1 | 0/20 | 0 |
| | | 3.1 | 0/19 | 0 |
| | | | | |
| Path3 | Uninf. CAM | < 2.5 | 0/20 | 0 |
| | A 489 | 3.2 | 18/18 | 100 |
| | del UL0 +H7 | 2.8 | 1/19 | 5 |
| | | 2.4 | 0/20 | 0 |

| | | | | |
|---|---|---|---|---|
| *) Inoculum dose is in Log₁₀ TCID₅₀ per animal (0.2 ml) | | | | |

### Example 4

### Animal trials to determine protection of vaccinated chickens against challenge infection with virulent ILTV and with highly pathogenic AIV

Further animal experiments were performed to test the suitability of UL0 deletion mutants of ILTV as life-virus vaccines, and as foreign-antigen expressing vectors that can protect chickens against other pathogens. To that purpose, 10 week old SPF chickens were immunized via eye drop with 10³ to 10⁴ plaque forming units (pfu) per animal of either ILTV ΔUL0, or ILTV ΔUL0-HA7. As expected, all animals survived immunization, and only few of them exhibited negligible clinical signs of ILT (Table 3). Two weeks after immunization, sera were collected from all animals and investigated for ILTV-, as well as for HA-specific antibodies. By indirect immunofluorescence tests (Lüschow et al., 2001, supra,), ILTV-specific antibodies were unequivocally detected in more than 70 % of the samples of both immunized groups (Table 3). In addition, all chickens immunized with ILTV ΔUL0-HA7 produced HA-specific antibodies as demonstrated by hemagglutinin inhibition tests (HAI; Alexander DJ, In: OIE Manual of Standards for Diagnostic Tests and Vaccines, 155-160, 1996) using AIV A/Italy/445/99 (H7N1) as antigen donor (Table 3).

After 25 days, subgroups of the vaccinated chickens (groups 1A, 2A), and non-immunized control animals (group 3) were challenged by intratracheal administration of 2 x 10⁵ pfu per animal of virulent wild type ILTV (A489). Mortality rates, and clinical symptoms of ILT were monitored and quantified as explained above (Example 3). The mean clinical scores of all individuals of each group were determined for days 2 to 12 after infection (Table 3). All non-immunized control animals exhibited severe signs of disease, which led to death in two out of four cases. In contrast, all vaccinated animals survived, and most of them remained healthy. These results clearly demonstrate that live-virus vaccination with UL0 deletion mutants of ILTV confers protective immunity against subsequent ILTV infection.

Two other subgroups (1B, 2B) of the chickens vaccinated with either ILTV ΔUL0-HA7, or ILTV ΔUL0 were challenged by intranasal administration of 108 embryo infectious doses (EID₅₀) per animal of the highly pathogenic AIV isolate A/Italy/445/99 (H7N1), which was also the donor of the HA gene expressed by ILTV ΔUL0-H7. Like non-immunized animals (not shown), all chickens vaccinated with ILTV ΔUL0 died within 4 days after AIV infection (Table 3). In contrast, all animals immunized with ILTV ΔUL0-HA7 survived the lethal dose AIV challenge, and the severity of disease was substantially reduced. Clinical signs of avian influenza were individually evaluated as follows:
score 0: animal healthy,
score 1: diarrhea, or edema, or animal depressed,
score 2: animal lies down and is unable to rise,
score 3: animal dead

The mean scores of each group were calculated for days 1 to 10 after challenge (Table 3). As determined by inoculation of chicken embryos with tracheal and cloacal swabs (Alexander DJ, supra, 1996), the AIV challenge virus was shed by many of the vaccinated animals, but only for a very limited time period. Thus, a singular live-virus vaccination of chickens with a ULO-negative ILTV recombinant expressing an H7 hemagglutinin is sufficient to induce a protective immunity against fowl plague caused by highly pathogenic AIV of the corresponding serotype.

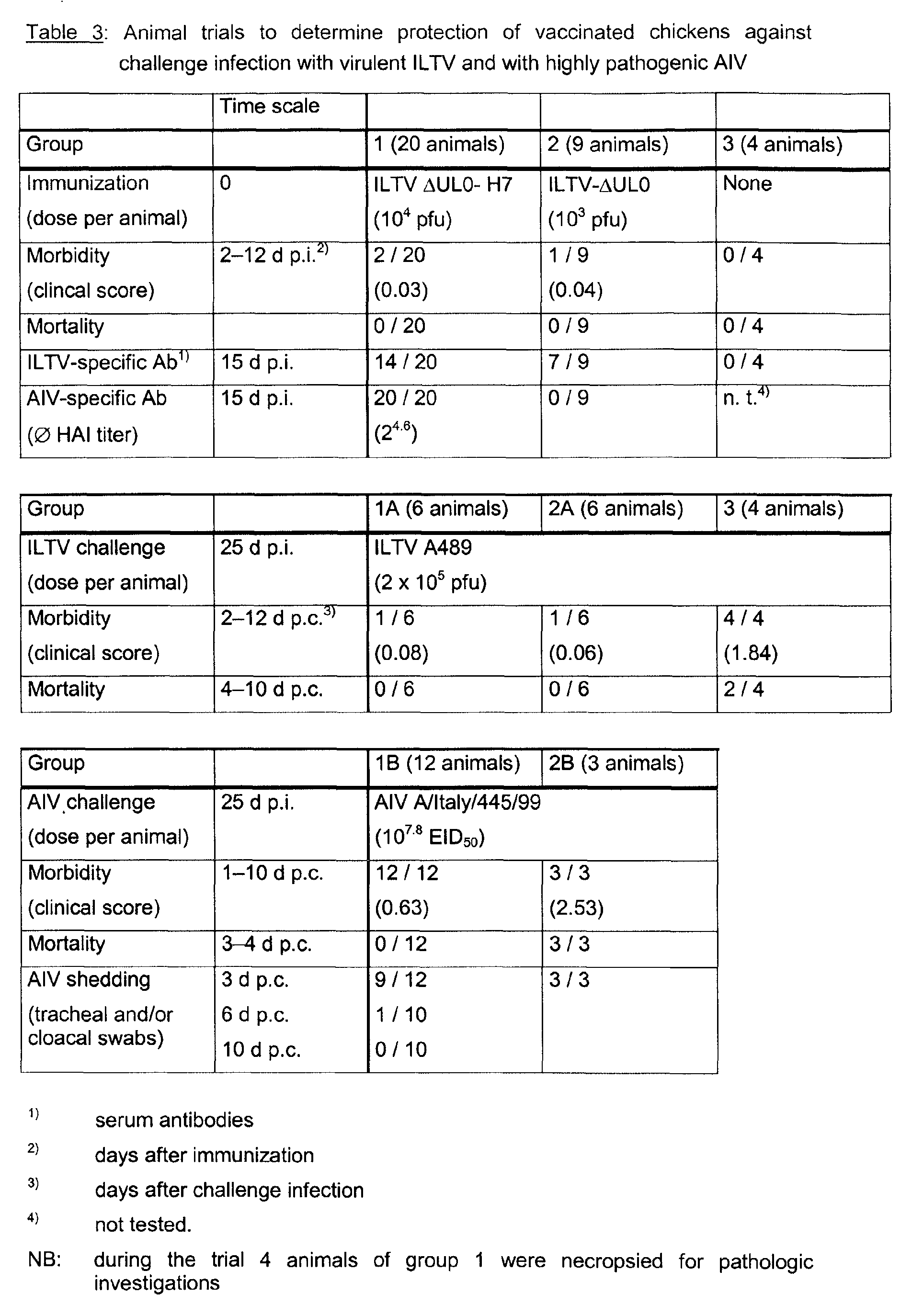

## Claims

1. A vaccine for the protection of poultry against disease caused by an avian pathogen comprising an attenuated infectious laryngotracheitis virus (ILTV) mutant and a pharmaceutically acceptable carrier or diluent, **characterized in that** the ILTV mutant is not able to express a native UL0 protein in an infected host cell as a result of a mutation in the UL0 gene.

2. A vaccine according to claim 1, **characterized in that** the mutation in the ULO gene is a deletion.

3. A vaccine according to claim 1, **characterized in that** the mutation in the ULO gene is an insertion of a heterologous nucleic acid sequence.

4. A vaccine according to claim 2, **characterized in that** the mutant comprises a heterologous nucleic acid sequence in place of the deletion.

5. A vaccine according to claims 3 or 4, **characterized in that** the heterologous nucleic acid sequence is under the control of an expression control sequence.

6. A vaccine according to claim 5, **characterized in that** the heterologous nucleic acid sequence encodes an antigen of an avian pathogen.

7. A vaccine according to claim 6, **characterized in that** the avian pathogen is avian influenza virus, Marek's disease virus, Newcastle disease virus, infectious bronchitis virus, turkey rhinotracheitis virus, E. coli, Ornithobacterium rhinotracheale or Mycoplasma.

8. A vaccine according to claim 5, **characterized in that** the heterologous nucleic acid sequence encodes an immunomodulator.

9. A cell culture infected with an ILTV mutant as defined in claims 1-8.

10. A process for the preparation of a vaccine for the protection of poultry against disease caused by an avian pathogen, **characterized in that** it comprises the step of mixing an ILTV mutant as defined in claims 1-8 with a pharmaceutically acceptable carrier or diluent.
